# EUROPEAN PATENT SPECIFICATION

(11) **EP 4 205 733 B1**
(45) Date of publication and mention of the grant of the patent: **23.10.2024**
(21) Application number: 22216685.2
(22) Date of filing: 27.12.2022
(51) Int. Cl.: A61K 31/05, A61K 31/122, A61K 31/352, A61K 31/80, A61K 36/9066, A61P 19/10

(54) **COMPOSITION FOR THE TREATMENT OF OSTEOPENIA AND OSTEOPOROSIS**
ZUSAMMENSETZUNG ZUR BEHANDLUNG VON OSTEOPENIE UND OSTEOPOROSE
COMPOSITION POUR LE TRAITEMENT DE L'OSTÉOPÉNIE ET DE L'OSTÉOPOROSE

(30) Priority: 29.12.2021 IT 202100032921
(43) Date of publication of application: 05.07.2023
(73) Proprietor: MIVELL S.R.L.S., 61032 Fano (PU) (IT)
(72) Inventor: MIGLIONICO, Leonardo, I-61032 FANO (Pesaro e Urbino) (IT); VALLI, Debora, I-60131 ANCONA (IT); SCARPA, Emanuele Salvatore, I-61122 PESARO (Pesaro e Urbino) (IT)
(74) Representative: Comoglio, Elena

(56) References cited:
- WO-A1-2014/138372
- US-A1- 2019 015 448
- US-B2- 10 632 101
- GRAY MICHAEL G ET AL: "Multiple Integrated Complementary Healing Approaches: Energetics & Light for bone", MEDICAL HYPOTHESES, EDEN PRESS, PENRITH, US, vol. 86, 24 November 2015 (2015-11-24), pages 18 - 29, XP029392319, ISSN: 0306-9877, DOI: 10.1016/J.MEHY.2015.10.030

## Description

The present invention relates to a composition for use as a dietary supplement or medicament in prevention and treatment of skeletal pathologies characterized by a reduction in bone mass, more specifically osteopenia and osteoporosis. Over the years there has been a remarkable increase in life expectancy in population of developed countries, which has resulted in a significant increase in the most common diseases and age-related conditions, including diabetes, osteopenia, osteoporosis, cardiovascular and neurodegenerative diseases. Osteoporosis is a skeletal disease generally associated with aging, characterized by a reduction in bone mass, measured as a bone mineral density (BMD) value and by an alteration of its architecture, which determines bone fragility and increased risk of fractures. Osteopenia is a systemic condition of the skeletal system, distinguished by a reduction in bone mineral density values, which is less severe than that observed in the case of osteoporosis. Noteworthy, the main osteogenic markers are the transcription factor Runt-related transcription factor 2 (RUNX2), alkaline phosphatase (ALP), collagen type 1 alpha 1 (COL1α1) and osteocalcin (OCN) **[Raisz, L.G., 2005].** Interestingly, it was shown that the natural molecule fisetin was able to increase the mRNA and protein levels of the osteogenic markers COL1α1 and RUNX2 in the pre-osteoblastic MC3T3-E1 cells, indicating the potential of this phytochemical for the osteoporosis treatment **[Xu, L. et al., 2021].** The capacity of the mesenchymal stem cells (MSC) to differentiate into osteoblasts depends on certain transcription factors that are expressed in different stages of bone mineralization. In a first phase RUNX2 orients the MSCs towards the osteoblastogenesis, by inhibiting their adipocytic and chondrocytic differentiation. Subsequently, the increase of the marker ALP favors the transition from pre-osteoblast to immature osteoblast, the stage in which the bone matrix proteins, including COL1α1, are produced. The immature osteoblasts, expressing high levels of osteopontin (OPN), will produce osteocalcin in the final stage of maturation into osteoblasts. The alteration of these different pathways, caused by dysfunction of the cellular microenvironment, can lead to the formation of bone marrow adipose tissue, decreasing the bone mineralization. The impoverishment of bone structure that arises from this event is further worsened by the fact that the newly formed adipocytes contribute to the secretion of a wide range of adipokines, which usually exert a protective role, but are also associated to inflammation, effectively increasing the risk to develop chronic inflammatory diseases. A chronic inflammatory state plays a pivotal role in the development of osteoporosis pathological condition. In fact, it is known that high levels of proinflammatory cytokines inhibit cell differentiation into osteoblasts and, together with other mediators of the inflammatory process, lead to an increase of the expression of the RANKL ligand which, after having bound to its RANK receptor expressed on pre-osteoclasts, favors their differentiation in mature osteoclasts. Therefore, the synthesis of new bone decreases while the bone resorption increases, thus leading to a reduction in bone mass during a chronic inflammatory state. Over the years various drugs have been developed with the purpose of prevention or treatment of osteoporosis, such as bisphosphonates or monoclonal antibodies. The bisphosphonate zoledronic acid is an effective drug used in the treatment of osteoporosis; this drug targets the acid phosphatase 5b, an enzyme involved in bone resorption mechanisms **[Mori, Y. et al., 2018].** On the contrary, it was shown that the zoledronic acid treatment was able to increase the expression levels of alkaline phosphatase and RUNX2 and to dose-dependently induce an increase of the catalytic activity of the ALP enzyme and of the mineralization process in the human bone marrow mesenchymal stromal cells (BM-MSCs) in vitro model, indicating ALP as a pivotal marker of the osteogenesis process **[Jin ZH et al., 2020].** However, the numerous adverse effects of conventional drug treatments highlighted over time have led to the need to identify natural substances that can produce the same beneficial effects, but without the toxic side effects. Interestingly, it was demonstrated that quercetin-3-O-β-D-galactopyranoside treatment of human BM-MSCs was able to stimulate the osteoblastogenesis process through the increase of ALP activity and also of RUNX2 and OCN levels **[Oh J.H. et al., 2020].** In order to satisfy the medical needs focused on innovative therapeutic treatments for osteoporosis and osteopenia, the present invention provides a composition with anti-inflammatory and pro-mineralizing actions as defined in the annexed claim 1. Additional features and benefits of the formulation of the invention are defined in the attached dependent claims. The composition of the present invention is characterized by the combination of the active ingredients orthosilicic acid, vitamin K2, polydatin, extract of *Curcuma longa* and quercetin. In the present description, the extract of *Curcuma longa* is sometimes referred to, for the sake of brevity, simply as "turmeric". Based on the data reported in the literature, orthosilicic acid and vitamin K2 have been chosen as substances with pro-mineralizing activity; polydatin, the extract of *Curcuma longa* and quercetin have been chosen as substances with anti-inflammatory activity. Ortho-silicic acid (H₂SiO₄) is a molecule that represents the major form of bioavailable silicon (Si) for both humans and animals **[Jurkic, L.M. et al., 2013].** Even though plant foods contain high levels of silicon, its bioavailability from these sources is questionable, due to poor solubility and poor absorption in the gastro-intestinal tract of the silicon. On the contrary, absorption studies indicate that the ortho-silicic acid is a main readily bioavailable source of silicon for humans; noteworthy, orthosilicic acid is able to increase osteoblastic differentiation, bone formation and BMD **[Jurkic, L.M. et al., 2013].** Numerous *in vitro* studies have shown the pro-mineralizing activity of the orthosilicic acid. In fact, in different human and murine cell lines, it was found that the treatment with orthosilicic acid causes an increase in the expression of the main osteogenic markers, such as ALP, RUNX2, OCN, COL1α1, OPN, thus promoting differentiation of osteoblasts and inhibition of osteoclastogenesis. Vitamin K2, and in particular the Menaquinone-7 (MK-7) inhibits bone resorption and promotes bone mineralization and then osteoblastogenesis. Some preclinical studies have highlighted the ability of the vitamin K2 to promote the differentiation of osteoblasts and the bone mineralization through the induction of ALP and RUNX2. Several clinical trials have shown that the treatment with vitamin K2 (MK7) improves BMD and determines a decrease in the levels of ucOC (decarboxylated osteocalcin) and an increase in cOC levels (carboxylated osteocalcin). Turmeric, and especially its compound curcumin, possesses an antioxidant action exerted through the inhibition of the superoxide radical, the hydrogen peroxide and the nitric oxide radical. In addition, curcumin increases the activity of many antioxidant enzymes, such as catalase, superoxide dismutase and glutathione peroxidase. Moreover, curcumin exerts an anti-inflammatory action, as it modulates the production of several pro-inflammatory interleukins, such as IL-6, IL-8, TNF-α and MCP-1, and reduces the activation of a set of enzymes involved in the inflammation process, such as iNOS and COX-2. Polydatin is a polyphenol belonging to the stilbene class and is the glycosylated form of resveratrol. Like curcumin, the polydatin exerts an antioxidant activity through the activation of the antioxidant pathway of SIRT1, the increase of expression of superoxide dismutase and of glutathione peroxidase. It has also been proven that polydatin improves osteoporosis condition through the inhibition of the MAPK signaling pathway involved in inflammation, apoptosis, cell proliferation and differentiation. It was demonstrated that in an *in vitro* model of osteoporosis (MC3T3-E1 cells), polydatin treatment was able to significantly increase the expression levels of osteogenic genes COL1α1, ALP, OCN, RUNX2 and to increase ALP protein levels and calcium levels **[Lin, Z. et al., 2021].** In addition, polydatin treatment decreased the protein levels of pro-inflammatory markers p-p38 and p-JNK and reduced the protein levels of p-ERK, which is involved in the RANKL-mediated osteoclast activation. These results have shown that the phytochemical polydatin, but not its aglycone resveratrol, possesses both anti-inflammatory and osteogenic biological activities **[Lin, Z. et al., 2021].** Numerous *in vivo* studies have highlighted the great potential of quercetin in maintaining the bone structure. Furthermore, several *in vitro* studies have confirmed the ability of quercetin to stimulate osteoblastogenesis and to inhibit the formation, proliferation and maturation of the osteoclasts. Quercetin also features an antioxidant and anti-inflammatory activity, exerted through the modulation of the activity of antioxidant enzymes, the inhibition of pro-inflammatory cytokines (TNF-α, IL-6, IL-1β, CRP) and the stimulation of anti-inflammatory cytokines (IL-10 and Arg-1). Even if the anti-inflammatory and pro-mineralizing activities of the single substances that characterize the composition of the present invention are known, the inventor has surprisingly observed a series of unexpected synergistic actions exerted by the combination of the substances cited above, both in reducing the expression levels of some cytokines implicated in inflammatory processes and in inducing the expression of some osteogenic markers. Such synergistic activities have been demonstrated through a series of experimental studies, illustrated in the examples of the present description. More specifically, Example 2 demonstrates that orthosilicic acid and vitamin K2 (Menaquinone 7) act synergistically in the induction of the expression of osteogenic markers RUNX2, COL1α1 and OCN in bone marrow mesenchymal stromal cells (BM-MSCs). Example 3 demonstrates that polydatin, curcumin and quercetin act synergistically in the reduction of the expression levels of the pro-inflammatory marker IL-1β in senescent BM-MSCs. Example 4 shows that the pro-mineralizing component of the composition of the invention (orthosilicic acid and vitamin K2) in combination with the anti-inflammatory component (curcumin, polydatin and quercetin) acts synergistically in inducing the expression of the osteogenic marker Col1α1 in young BM-MSCs cells. Furthermore, the combination of these five substances can synergistically increase the expression levels of the osteogenic marker ALP in senescent BM-MSCs, demonstrating the pro-mineralizing effect of this formulation.

The described synergistic activities make the composition of the invention effective against the states of osteopenia and osteoporosis, which are characterized by a decrease in synthesis of new bone tissue and by the concomitant increased bone resorption, caused also by an important inflammatory component. In a preferred embodiment, the composition of the invention is a preparation for oral use, more preferably an oral pharmaceutical preparation in solid or liquid form, such as a tablet, soft or hard capsule, pill, a powder, granulate, or solid form with a controlled release. These pharmaceutical forms are prepared according to known conventional methods in the pharmaceutical field, such as those described in **[**Remington: The Science and Practice of Pharmacy, 23rd edition (2020), Adeboye Adejare ed**.],** using suitable excipients, vehicles and/or bases per se known in art. In another preferred embodiment, the composition of the invention comprises, for dosage unit:
- from 20 to 100 mg of polydatin;
- from 20 to 300 mg of quercetin;
- 50 to 500 mg of *Curcuma longa* extract;
- 50 to 200 µg of Vitamin K2 (Menaquinone-7);
- 5 to 50 mg of orthosilicic acid.

The present description also includes the following dosage ranges of **polydatin:** 20 to 25 mg, 25 to 30 mg, 30 to 35 mg, 35 to 40 mg, 40 to 50 mg, 50 to 60 mg, 60 to 70 mg, 70 to 80 mg, 80 to 90 mg and 90 to 100 mg. The present description also includes the following dosage ranges of **quercetin:** 20 to 30 mg, 30 to 40 mg, 40 to 50 mg, 50 to 55 mg, 55 to 60 mg, 60 to 65 mg, 65 to 70 mg, 70 to 80 mg, 80 to 90 mg, 90 to 100 mg, 100 to 110 mg, 110 to 120 mg, 120 to 130 mg, 130 to 140 mg, 140 to 150 mg, 150 to 200 mg, 200 to 250 mg, 250 to 300 mg. The present description also includes the following dosage ranges of the extract of ***Curcuma longa*:** 50 mg to 100 mg, 100 mg to 150 mg, 150 mg to 160 mg, 160 mg to 170 mg, 170 mg to 180 mg, 180 mg to 190 mg, 190 mg to 200 mg, 200 mg to 210 mg, 210 mg to 220 mg, 220 mg to 230 mg, 230 mg to 240 mg, 240 mg to 250 mg, 250 mg to 300 mg, 300 mg to 350 mg, 350 mg to 400 mg, 400 mg to 450 mg, 450 mg to 500 mg. The present description also includes the following dosage ranges of **Vitamin K2** (Menaquinone-7): 50 to 60 µg, 60 to 70 µg, 70 to 80 µg, 80 to 90 µg, 90 to 100 µg, 100 to 110 µg, 110 to 120 µg, 120 to 130 µg, 130 to 140 µg, 140 to 150 µg, 150 to 160 µg, 160 to 170 µg, 170 to 180 µg, 180 to 190 µg, 190 to 200 µg. The present disclosure also includes the following **orthosilicic acid** dosage ranges: from 5 to 10 mg, 10 to 15 mg, 15 to 20 mg, 20 to 25 mg, 25 to 30 mg, 30 to 35 mg, 35 to 40 mg, 40 to 45 mg, 45 to 50 mg. The dosage ranges described above for polydatin, quercetin, *Curcuma longa* extract, vitamin K2 (menaquinone-7) and orthosilicic acid can be combined with each other. In another preferred embodiment, the formulation of the invention also includes vitamin D3. Vitamin D3 treatment increases the BMD, leading to a reduction of the risk of fractures and is also able to increase the calcium absorption in the intestine **[Christakos S. 2012].** The following examples are illustrative and do not limit the scope of the invention, as defined in the annexed claims.

### EXAMPLES

### Example 1: Example formulation

BlastiMin Complex^{®} is a food supplement based on:
- OxiDef^{®} (curcumin, polydatin and quercetin)
- Orthosilicic acid
- Vitamin K2 (Menaquinone-7)
- Vitamin D3

Pharmaceutical form: gastro-protected tablets.

Ingredients: turmeric extract titrated at 95% in total curcuminoids (*Curcuma Longa L.*, rhizome and essential oils), polydatin (*Polygonum Cuspidatum* root), quercetin (*Sophora japonica L.*), orthosilicic acid, vitamin K2 (Menaquinone-7) and vitamin D3. Anti-caking agents: microcrystalline cellulose, silicon dioxide and salts of fatty acid magnesium.

**Table 1: Average contents per daily dose**

| **Active substance** | **Content for 1 capsule** | **Reference values** |
|---|---|---|
| Extract of *Curcuma longa* (95% curcuminoids, of which 70% curcumin) | 200 mg | |
| Quercetin | 60 mg | |
| Polydatin | 30 mg | |
| Orthosilicic acid | 25 mg | |
| Vitamin K2 | 150 µg | |
| Vitamin D3 | 50 µg | 2000 I.U. |

The recommended dose is 1 tablet a day.

### Example 2: Study of the pro-mineralizing effects of the substances

The purpose of this study is to evaluate the pro-mineralizing activity of orthosilicic acid and vitamin K2 of the composition, as well as the synergy between them, by measuring the ability to induce the expression of various osteogenic markers in BM-MSCs cells.

### Materials and methods

### 2.1 Tested compounds

The ingredients tested with the aim of highlighting a pro-mineralizing activity are orthosilicic acid (ingredient A) and vitamin K2 (Menaquinone MK-7) (ingredient B). For this study, both standards were purchased from Sigma Aldrich: the vitamin K2 (Menaquinone-7) (PHR2363) and the sodium metasilicate (307815), which has been used to obtain the orthosilicic acid.

### 2.2 Solubility test

Before proceeding with the cellular assays, the two ingredients have been tested for their solubility in several solvents, with the aim of identifying the best solubilizing agent **(Table 2).**

**Table 2. Solubility test results.**

| **Substances** | **PBS** | **DMSO** | **Organic solvents** | **Cremophor** |
|---|---|---|---|---|
| *Sodium metasilicate* | Soluble | Not tested | Not tested | Not tested |
| *Vitamin K2* | Not soluble | Not soluble | Not soluble | Soluble |

### Protocol of solubilization of sodium metasilicate with PBS

It has been observed that at high concentrations orthosilicic acid is a very unstable molecule. For this reason, in order to obtain the desired concentration of orthosilicic acid, an intermediate dilution was prepared in 10 mL of PBS, then rapidly diluted 1: 1000.

### Vitamin K2 (Menaquinone-7) solubilization protocol in Cremophor

Regarding the preparation of the vitamin K2 solution, 100 mg of Polyoxyethylene hydrogenated oil (HCO-60) Nikkol (trade name: Cremophor) are brought to 60°C. The substance of interest is weighed and brought to a total weight of 100 mg by adding glycerol. The two solutions are then combined and brought to a final volume of 1 mL with dH₂0.

### 2.3 Cell cultures

The experiments were conducted on BM-MSCs cells, obtained from 30-year-old Caucasian donor (Promocell). The BM-MSCs were grown in maintenance medium MSC-GM2 (PromoCell) with the addition of Supplement Mix and Penicillin-Streptomycin 1%, at 37°C with 5% CO₂. For the osteogenic differentiation the osteogenic medium (Osteogenic Medium, PromoCell) was added with specific supplements and Penicillin-Streptomycin 1%. We evaluated the proliferative capacity and the differentiating capacity of this cell model using the pro-differentiating terrain and cellular senescence condition was characterized (data not shown).

### 2.4 Cytotoxicity assays

The MTT assay is a colorimetric test of cytotoxicity that allows to evaluate the cellular viability based on the efficiency of mitochondrial respiration. For assay setup, cells are seeded homogeneously in 96-wells plates at a density of 5000 cells/cm² and incubated at 37°C with 5% CO₂. The next day the cells were treated with the concentrations reported in **Table 3,** with the aim of identifying the maximum non-cytotoxic concentration for subsequent analyses. Since 7 to 21 days are needed to achieve osteogenic differentiation **[Zhou et al., 2021],** the treatments were carried out for 24h, 72h and 96h, up to a maximum of 10 days. At the end of the treatment, the MTT solution (5 mg/ml) in PBS was added and the cells were incubated for 3 hours at 37°C with 5% CO₂. Then, after a short wash in PBS, 10 µL of DMSO have been added to obtain the solubilization of formazan crystals. The absorbance values at 540 nm of the samples have been used to assess the cell viability, expressed as a percentage relative to the negative control, i.e. to untreated cells.

**Table 3. Range of concentrations of orthosilicic acid and vitamin K2 (MK-7) tested for the MTT assays.**

| **Orthosilicic acid [µg/ml]** | **Vitamin K2 [µg/ml]** | **Cremophor [µg/ml]** |
|---|---|---|
| 0 | 0 | 0 |
| 6.1 | 0.05 | 0.05 |
| 9.15 | 0.06 | 0.06 |
| 12.2 | 0.2 | 0.2 |
| 15.25 | 0.4 | 0.4 |
| 18.3 | 0.81 | 0.81 |
| | 1.62 | 1.62 |
| | 3.2 | 3.2 |
| | 6.49 | 6.49 |

### 2.5 RTqPCR for the evaluation of the mRNA levels of osteogenic markers

The pro-mineralizing activity of individual ingredients orthosilicic acid and vitamin K2 and their combination was determined according to the modulation of the expression levels of the osteogenic markers RUNX2, ALP, COL1α1 and OCN, analyzing the levels of their mRNAs. For assay setup, young BM-MSCs were seeded in 12-wells plates, with a density of 3000 cells/cm² and treated with maintenance medium, added with the non-toxic concentrations of the substances under consideration, used individually or in combination. The treatments were carried out for 7 and 14 days, at the end of which, after a short washing in PBS, the cells were lysed directly on plate, with a lysis buffer present in the RNA extraction kit (Norgen Biotek Corporation, Canada). After the extraction process and the quantification with the Nanodrop instrument, RNA was reverse transcribed using the Takara Bio Inc. kit. The obtained cDNAs have been used to perform the RTqPCR assays.

**Table 4. List of the primers used for the execution of the RTqPCR assays with SYBR-Green.**

| Gene | Sequence | Sequence ID |
|---|---|---|
| IPO8-F | CGTTCCTCCTGAGACTCTGC | 1 |
| IPO-8 R | GAATGCCCACTGCATAGGTT | 2 |
| COL1α1-F | CCAAATCTGTCTCCCCAGAA | 3 |
| COL1α1-R | TCAAAAACGAAGGGGAGATG | 4 |
| ALP-F | GAGAAGCCGGGACACAGTTC | 5 |
| ALP-R | CCTCCTCAACTGGGATGATGC | 6 |
| RUNX2-F | GCGGTGCAAACTTTCTCCAG | 7 |
| RUNX2-R | TGCTTGCAGCCTTAAATGACTC | 8 |
| OCN-F | CTCACACTCCTCGCCCTATTG | 9 |
| OCN-R | GCTTGGACACAAAGGCTGCAC | 10 |

### 2.6 Western blotting for the evaluation of the protein levels of osteogenic markers

The pro-mineralizing activity of the tested substances was also evaluated through the analysis of the modulation of protein levels. Western blotting is a technique used for the identification of specific proteins of interest present within a protein blend, through the use of specific antibodies. The method used for protein separation is the SDS-PAGE, followed by the transfer on nitrocellulose membrane by electroblotting. After a phase of blockage of the non-specific sites of the membrane, the following primary antibodies were used: GAPDH monoclonal antibody (Cell Signaling); OCN polyclonal antibody (Biorbyt); RUNX2 monoclonal antibody (Cell Signaling); COL1α1 monoclonal antibody (Cell Signaling). Furthermore, the method used for the development of the membrane was the chemiluminescence (ECL, Biorad) and the detection was carried out with the UVITEC Alliance tool (Cambridge), equipped with an acquisition system of high resolution images.

### 2.7 Statistical analysis

Data were expressed as mean ± SD (standard deviation). Student's t-test was used to determine the p values. GraphPad (Prism software) was used for statistical analysis. p<0.05 was considered statistically significant.

### 2.8 Results

### Evaluation of the cytotoxicity of tested compounds

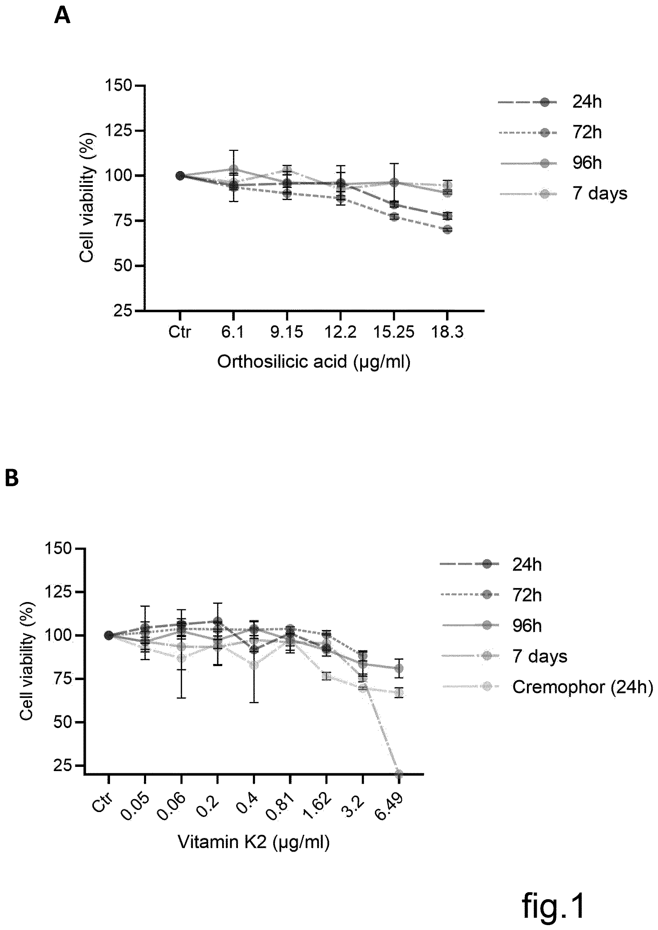

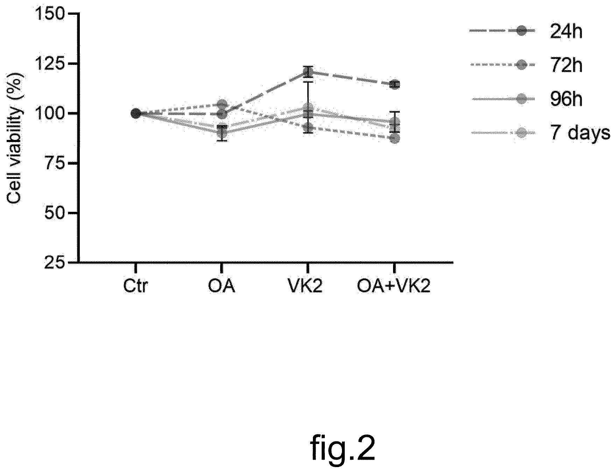

The results related to cell viability (MTT test) after the treatments with different concentrations of ingredient A (orthosilicic acid) and ingredient B (vitamin K2) in the culture medium at different times are shown in **Figure 1****.** For the determination of cell viability of the cells treated with the Cremophor vehicle, **Figure 1** shows only the results related to the 96h treatment. In subsequent experiments, the BM-MSCs have been treated with 9.15 µg/ml of orthosilicic acid **(****Figure 1A****)** and 0.06 µg/ml of vitamin K2 **(****Figure 1B****).** With the aim of studying the synergy between the two ingredients, it was necessary to evaluate the possible cytotoxicity of the combination. **Figure 2** shows the graph related to cell viability of the cells treated with individual ingredients and with their combination, expressed as a percentage compared to the untreated control. As it can be seen from the graph, the association of ingredients A and B does not cause a decrease in vitality compared to the untreated control; in addition, the compounds under examination show no particularity toxicity. **Table 5** shows the concentrations used for individual treatments and for the combination of the two ingredients.

**Table 5. Concentrations (µg/ml) tested for the individual ingredients and their combination.**

| **Ingredient** | **Substance** | **Concentration [µg/ml]** |
|---|---|---|
| A | Orthosilicic acid | 9.15 |
| B | Vitamin K2 | 0.06 |
| A+B | Orthosilicic acid+Vitamin K2 | 9.15+0.06 |

### Evaluation of the pro-mineralizing activity of tested compounds by RTqPCR assays

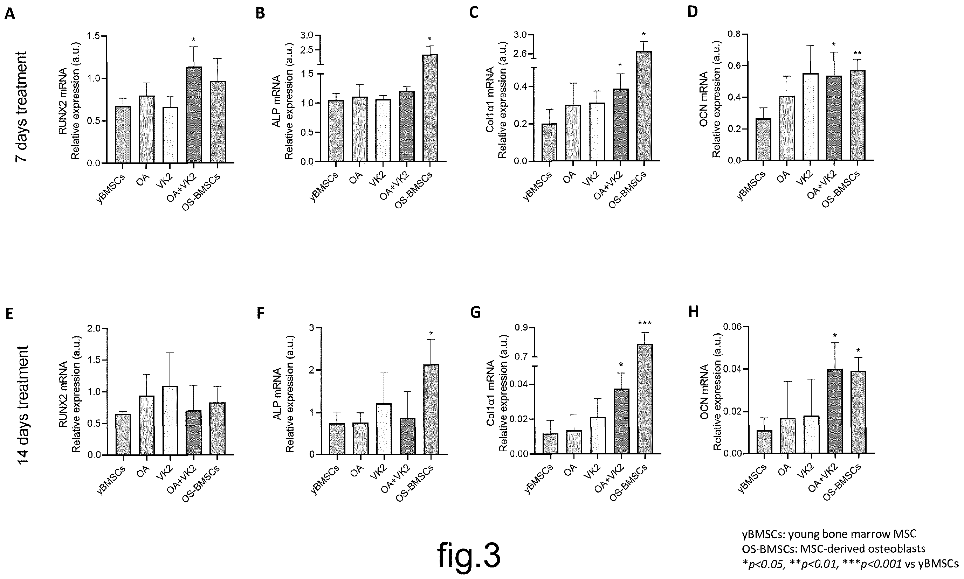

The ability of the individual ingredients to stimulate mineralization and the synergy between them was assessed as the ability to modulate the gene expression of the main osteogenic markers RUNX2, ALP, COL1α1 and OCN. The young BM-MSCs cells were grown in maintenance medium supplemented with 9.15 µg/ml of ingredient A (orthosilicic acid), 0.06 µg/ml of ingredient B (vitamin K2) and their combination (A+B) for 7 and 14 days. The cells grown in maintenance medium without the addition of the substances were used as a negative control. **Figure 3** shows the graphs related to the gene expression of the main osteogenic markers RUNX2, ALP, COL1α1 and OCN in young BM-MSCs treated for 7 days and 14 days with 9.15 µg/ml of orthosilicic acid and 0.06 µg/ml of vitamin K2. The asterisks (*) indicate significance values when compared with control (yBMSCs); * p< 0.05, ** p< 0.01, ***p<0.001 vs control, Student T-test. The results show that after 7 days of treatment the combination of the two ingredients determine a statistically significant increase of the expression of the osteogenic markers RUNX2 **(****Figure 3A****),** COL1α1 **(****Figure 3C****)** and OCN **(****Figure 3D****),** while, after 14 days, a statistically significant increase of the expression levels of COL1α1 **(****Figure 3G****)** and OCN **(****Figure 3H****)** was observed; these markers are related to the second phase of bone mineralization **[Rutkovskiy et al., 2016; Infante et al, 2018].** Noteworthy, the combination of orthosilicic acid and vitamin K2 induced a **synergistic** increase of RUNX2 mRNA levels after 7 days treatment and also of COL1α1 and OCN mRNA levels after 14 days treatment **(****Figure 3****).**

### Evaluation of the pro-mineralizing activity of tested compounds by Western blotting

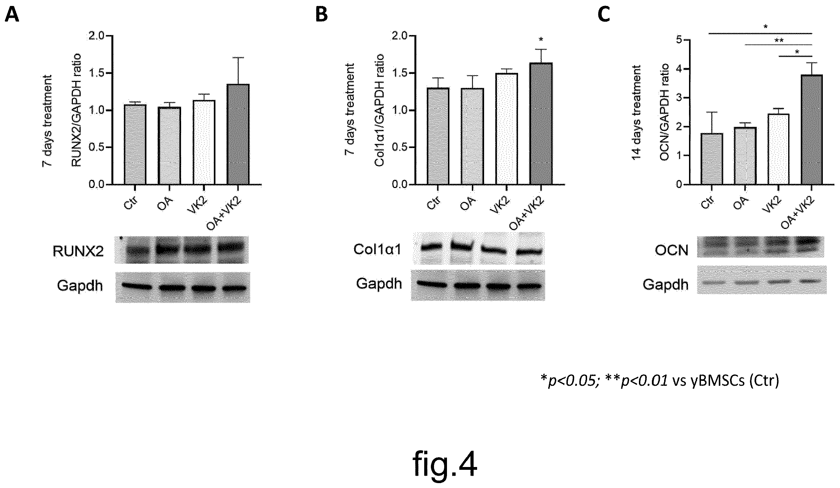

We also evaluated the protein levels of the main osteogenic markers RUNX2, Col1α1 and OCN, through the Western Blotting technique. **Figure 4** shows the protein levels of the main osteogenic markers after 7 days (RUNX2 and COL1α1) and 14 days (OCN) of treatment. The densitometric analysis of the blots is the result of the mean of three independent experiments; *p<0.05; **p<0.01, Student T-test. The statistically significant and synergistic increase in Col1α1 protein levels after 7 days treatment **(****Figure 4B****)** and in OCN protein levels after 14 days treatment **(****Figure 4C****),** induced by the combination of the two compounds, confirms the results observed with the analysis of the mRNA levels of these markers, indicating once again the effective pro-osteogenic and pro-mineralizing activity of the combination of orthosilicic acid and vitamin K2.

### 2.9 Conclusions

The obtained results show that the association of orthosilicic acid and vitamin K2 of the composition of the invention causes an increase of both mRNA and protein levels of osteogenic markers, thus highlighting the pro-mineralizing activity. Specifically, orthosilicic acid and vitamin K2 significantly increase the mRNA levels of the osteogenic markers RUNX2, COL1α1 and OCN after 7 days of treatment and also of COL1α1 and OCN after 14 days of treatment in BM-MSCs cells. In addition, the treatment with the combination of orthosilicic acid and vitamin K2 induced a significant increase of Col1α1 protein levels after 7 days and of OCN protein levels after 14 days of treatment in BM-MSCs cells.

### Example 3: study of the anti-inflammatory effects of the substances

The purpose of the study is to evaluate the anti-inflammatory activity of the individual ingredients of the composition (curcumin, polydatin, quercetin) of the invention and the possible synergy between them, measuring the ability to reduce expression levels of some cytokines involved in the inflammatory processes in BM-MSCs cells.

### Materials and methods

### 3.1 Tested compounds

The ingredients tested to highlight an anti-inflammatory activity are Turmeric (ingredient C), polydatin (ingredient D) and quercetin (ingredient E). For this study the following standards were used: Turmeric (A218580100, Carlo Erba), polydatin (P1878, TCI), quercetin (P0042, TCI).

### 3.2 Solubility test

All compounds have been preventively dissolved in dimethyl sulfoxide (DMSO) and subsequently diluted in complete medium, so the percentage of solvent was not greater than 0.1%, a concentration known to be non-toxic.

### 3.3 Cell cultures

The experiments were conducted on mesenchymal stromal cells obtained from the bone marrow of a 30-year-old Caucasian donor: BM-MSCs (Promocell). BM-MSCs were cultured in medium maintenance MSC-GM2 (PromoCell) with the addition of Supplement Mix and Penicillin-Streptomycin 1%, in humid atmosphere at 37°C, with 5% CO₂. The proliferative capacity of this cell model was evaluated and characterized and the cellular senescence was assessed using p16, p21 expression and beta-galactosidase activity (data not shown). The senescent mesenchymal stromal cells were used to highlight a possible anti-inflammatory effect of ingredients C, D and E. The senescent MSCs are, in fact, characterized by an inflammatory chronic state confirmed by the increase of the expression levels of pro-inflammatory cytokines (II,-1β, IL-6, IL-8 and MCP-1), when compared to young BM-MSCs.

### 3.4 Cytotoxicity assay

The MTT assay is a colorimetric test of cytotoxicity that allows to evaluate the cellular viability based on the efficiency of mitochondrial respiration. For assay setup, cells have been seeded homogeneously in 96-wells plates at a density of 5000 cells/cm² and incubated at 37°C, with 5% CO₂. The next day the cells were treated for 24, 48 and 72 hours with the concentrations reported in **Table 6,** in order to identify the highest non-cytotoxic concentrations to be used for subsequent analyses. At the end of the treatment, MTT solution was added (5 mg/ml) in PBS for 3 hours, at 37°C, with 5% CO₂. Then, after a short wash in PBS, 10 µL of DMSO were added for the solubilization of formazan crystals. The absorbance values at 540 nm of the samples have been used to assess the cell viability, expressed as a percentage relative to the negative control, i.e. to untreated cells.

**Table 6. Concentrations of curcumin, polydatin and quercetin used for MTT assays.**

| **Curcumin [µg/ml]** | **Polydatin [µg/ml]** | **Quercetin [µg/ml]** |
|---|---|---|
| 0 | 0 | 0 |
| 0.23 | 2.43 | 0.03 |
| 0.46 | 4.87 | 0.07 |
| 0.92 | 9.75 | 0.15 |
| 1.84 | 19.50 | 0.30 |
| 3.68 | 39.03 | 0.60 |
| 7.36 | | |
| 14.72 | | |

### 3.5 RTqPCr for the evaluation of the mRNA levels of inflammatory cytokines

The anti-inflammatory activity of the individual ingredients and the synergy between them have been assessed by the measurement of mRNA levels of the cytokines involved in inflammatory processes: IL-1β, IL-6, IL-8 and MCP-1 **[Lencel et al., 2011; Mulholland et al., 2019].** For the assay setup, the pre-senescent cells (P13 or P14) were plated in 12-well plates, with a density of 10⁴ cells/cm² and treated with maintenance medium, added with the maximum not-toxic concentrations of the tested substances, used individually and in combination. The treatments were carried out for 48h and then, after a short wash in PBS, cells were lysed directly in the plate, with the lysis buffer of the total RNA extraction kit (Norgen Biotek Corporation, Canada). After the process of extraction and quantization, the RNA was reverse transcribed using Takara Bio Inc. kit. The obtained cDNAs have been used to perform RTqPCR assays. **Table 7** shows the list of primers used for the RTqPCR experiments, utilizing the SYBR-Green (Takara Bio Inc.).

**Table 7. List of the sequence of primers used for RTqPCR assays.**

| Gene | Sequence | Sequence ID |
|---|---|---|
| IPO8-F | CGTTCCTCCTGAGACTCTGC | 1 |
| IPO8-R | GAATGCCCACTGCATAGGTT | 2 |
| IL-1β-P | AGATGATAAGCCCACTCTACAG | 11 |
| IL-1β-R | ACATTCAGCACAGGACTCTC | 12 |
| IL-6-F | TGCAATAACCACCCCTGACC | 13 |
| IL-6-R | GTGCCCATGCTACATTTGCC | 14 |
| IL-8-F | GGACAAGAGCCAGGAAGAAA | 15 |
| IL-8-R | CCTACAACAGACCCACACAATA | 16 |
| MCP-1-F | GGCTGAGACTAACCCAGAAAAG | 17 |
| MCP-1-R | GTGCCCATGCTACATTTGCC | 18 |

### 3.6 Western blotting for the evaluation of the protein levels of inflammatory markers

The anti-inflammatory activity of the substances in examination was evaluated also by analyzing the protein levels of p-p38, p-NF-kB and STAT3. Western blotting is a technique used for the identification of specific proteins of interest present within a protein blend, through the use of specific antibodies. The method used for protein separation is the SDS-PAGE, followed by the transfer on nitrocellulose membrane by electroblotting. After a phase of blockage of the non-specific sites of the membrane, the following primary antibodies were used: p-p38 monoclonal antibody (Cell Signaling); β-Actin monoclonal antibody (Cell Signaling); p-NF-kB monoclonal antibody (Cell Signaling); STAT3 monoclonal antibody (Cell Signaling). Furthermore, the method used for the development of the membrane was the chemiluminescence (ECL, Biorad) and the detection was carried out with the UVITEC Alliance tool (Cambridge), equipped with an acquisition system of high resolution images.

### 3.7 Statistical analysis

Data were expressed as mean ± SD (standard deviation). Student's t-test was used to determine the p values. GraphPad (Prism software) was used for statistical analysis. p<0.05 was considered statistically significant.

### 3.8 Results

### Evaluation of the cytotoxicity of tested compounds

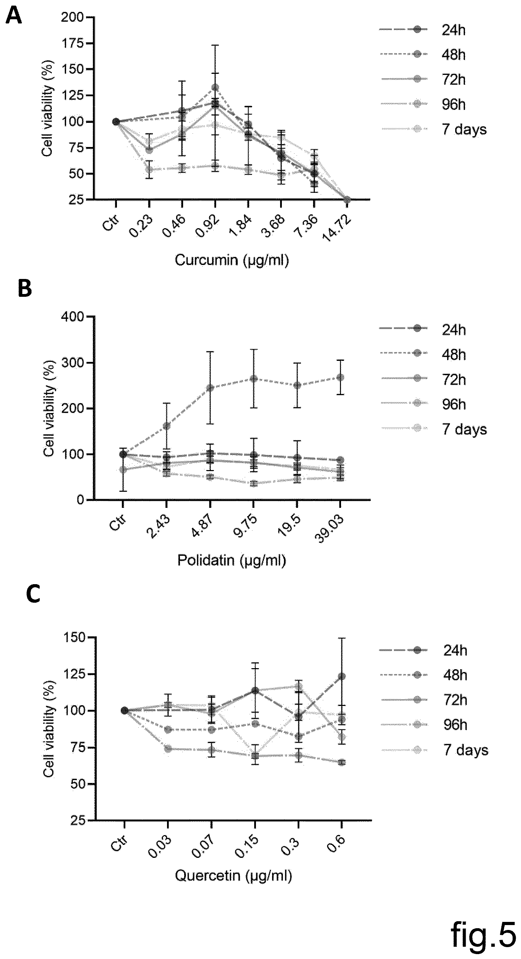

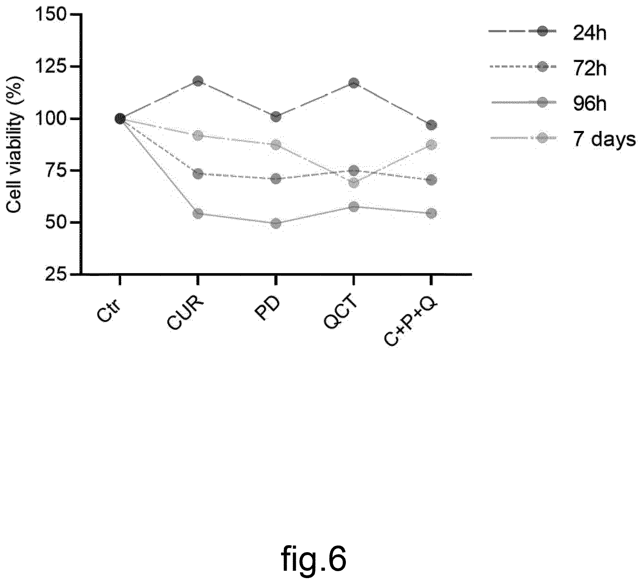

The results related to cell viability (MTT test) are reported in **Figure 5****,** which shows the cell viability after the treatments with different concentrations of ingredients C (curcumin), D (polydatin) and E (quercetin) in culture medium at different times. **Figure 5** shows that the compounds under consideration are not particularly toxic. In subsequent experiments, the senescent BM-MSCs were treated with 0.36 µg/ml of ingredient C (curcumin) **(****Figure 5A****),** with 3.9 µg/ml of ingredient D (polydatin) **(****Figure 5B****)** and with 0.15 µg/ml of ingredient E (quercetin) **(****Figure 5C****).** With the aim of studying the synergy between the three ingredients (C+D+E), it was necessary to evaluate the possible cytotoxicity of their combination. **Figure 6** shows the graph related to cell viability after the treatments with the individual ingredients and with their combination, expressed as a percentage compared to the untreated control cells. As it can be seen from the results, the combination of the ingredients does not determine a significant decrease in cell vitality. In **Table 8** the concentrations used for individual treatments and for the combination of the three ingredients are reported.

**Table 8. Concentrations (µg/ml) tested for the individual ingredients and for their combination for MTT assays.**

| **Ingredient** | **Substance** | **Concentration [µg/ml]** |
|---|---|---|
| C | Curcumin | 0.36 |
| D | Polydatin | 3.90 |
| E | Quercetin | 0.15 |
| C+D+E | Curcumin+Polydatin+Quercetin | 0.36+3.90+0.15 |

### Evaluation of the anti-inflammatory activity of the tested compounds by RTqPCR

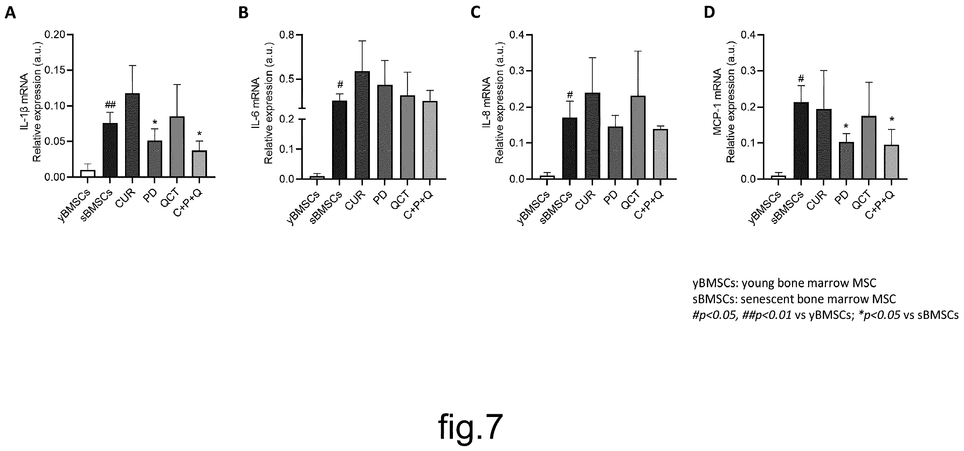

The anti-inflammatory activity of the individual ingredients and the synergy between them was evaluated as the ability to reduce the expression levels of pro-inflammatory cytokines in senescent BM-MSCs. The pre-senescent cells were cultured in maintenance medium supplemented with 0.36 µg/ml of ingredient C, 3.9 µg/ml of ingredient D, 0.15 µg/ml of ingredient E or their combination for 48h. Cells grown in medium maintenance without substances were used as a negative control. **Figure 7** shows the graphs relative to the gene expression of IL-1β, IL-6, IL-8 and MCP-1 in senescent BM-MSCs, treated for 48h with individual ingredients or their combination. The asterisks (*) indicate significance towards the sample of senescent BM-MSCs (sBMSCs), the symbol # indicates the significance when compared with the sample of young BM-MSCs (yBMSCs). *p<0.05; #p<0.05; ##p<0.01, Student T-test. The obtained results show that the combination of curcumin, polydatin and quercetin determines a significant decrease of the mRNA levels of IL-1β **(****Figure 7A****)** and of MCP-1 **(****Figure 7D****).** Noteworthy, the combination of curcumin, polydatin and quercetin induced a **synergistic** decrease of IL-1β mRNA levels in senescent BM-MSCs **(****Figure 7A****).** Interestingly, the polydatin treatment was also able to significantly decrease the expression levels of the pro-inflammatory markers IL-1β **(****Figure 7A****)** and MCP-1 **(****Figure 7D****).** On the contrary, neither the individual treatments nor the combination treatment were able to significantly decrease the expression levels of the pro-inflammatory markers IL-6 and IL-8 **(****Figure 7B**,**C**).

### Evaluation of the anti-inflammatory activity of the tested compounds by Western blotting

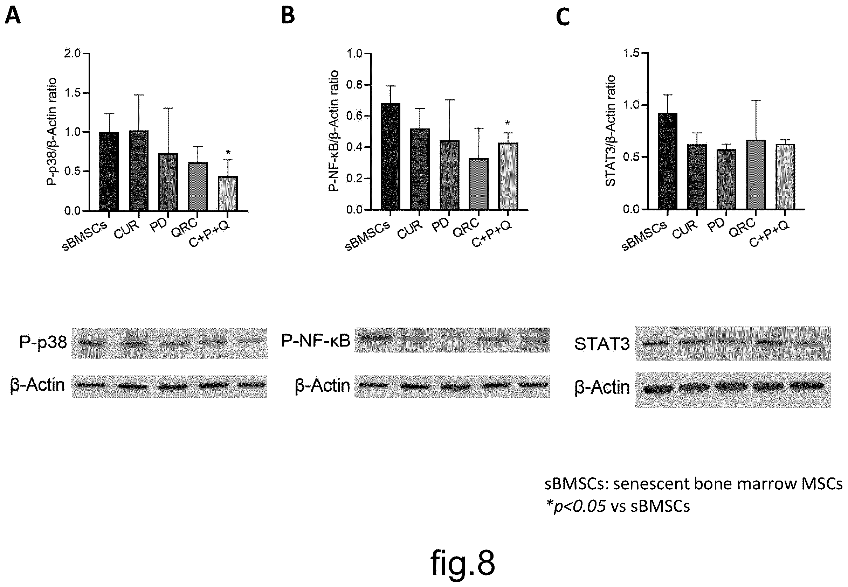

The anti-inflammatory activity of these compounds was also evaluated by analyzing the protein levels of the pro-inflammatory markers P-p38, P-NF-kB and STAT3, as shown in **Figure 8****.** The combination of curcumin+polydatin+quercetin was able to significantly decrease the P-p38 protein levels **(****Figure 8A****).** Noteworthy, p38 regulates the expression of some pro-inflammatory cytokines and interleukins such as TNFα, IL-1β, IL-6, IL-8, IL-4, IL-13 among others inflammatory mediators such as MCP-1, interferon-y and COX-2 **[Bachstetter et al., 2010; Madkour et al., 2021].** Moreover, the combination of curcumin, polydatin and quercetin induced a significant decrease of the protein levels of the pro-inflammatory transcription factor P-NF-kB **(****Figure 8B). Figure 8C** shows that both the individual treatments and the combination of these phytochemicals induced a slightly (but not significant) decrease of the protein levels of the pro-inflammatory transcription factor STAT3. The statistically significant decrease of P-p38 and P-NF-kB protein levels obtained in the cells treated with the combination of curcumin, polydatin and quercetin demonstrates the anti-inflammatory activity of the combination of these three natural substances.

### 3.9 Conclusions

The obtained results show that the association of curcumin, polydatin and quercetin of the composition of the invention determines a considerable and significant decrease of the expression levels of inflammatory cytokines IL-1β and MCP-1 in senescent BM-MSCs, thus highlighting its anti-inflammatory activity. Furthermore, these natural substances are also able to significantly decrease the protein levels of both P-p38 and P-NF-kB, which regulate the expression of several molecules involved in the development of a chronic inflammatory state.

### Example 4: study of the synergy between the anti-inflammatory and the pro-mineralizing components of the invention

The purpose of this study is to evaluate the synergistic activity between the pro-mineralising component (orthosilicic acid+vitamin K2) and the anti-inflammatory component (curcumin+polydatin+quercetin) of the composition of the invention, by means of measurement of the ability to induce expression of osteogenic markers in young and senescent BM-MSCs cells and to decrease the expression of pro-inflammatory markers in senescent BM-MSCs.

### Materials and methods

### 4.1 Tested compounds

The two components tested with the aim of highlighting a synergistic activity are: pro-mineralising component (orthosilicic acid and vitamin K2) and anti-inflammatory component (curcumin, polydatin and quercetin). For the study, we used the following standards: Vitamin K2/Menaquinone-7 (PHR2363, Sigma Aldrich), curcuma (A218580100, Carlo Erba), polydatin (P1878, TCI), quercetin (P0042, TCI), while in order to obtain orthosilicic acid, sodium metasilicate (307815, Sigma Aldrich) was used.

### 4.2 Solubility test

Sodium metasilicate was dissolved in PBS, Menaquinone-7 in Cremophor, while all the other ingredients have been prepared beforehand and dissolved in dimethyl sulfoxide (DMSO) and subsequently diluted in complete medium, so that the percentage of solvent was not greater than 0.1%, a non-toxic concentration.

### Sodium metasilicate solubilization protocol in PBS

With the aim of obtaining the desired concentration of orthosilicic acid, an intermediate dilution was first prepared in 10 mL of PBS, rapidly diluted 1:1000.

### Solubilization protocol of Vitamin K2/Menaquinone-7 in Cremophor

Regarding vitamin K2 preparation, 100 mg of Polyoxyethylene hydrogenated oil (HCO-60) Nikkol (trade name: Cremophor) are brought to 60°C. The substance of interest is weighed and brought to a total weight of 100 mg by adding glycerol. The two solutions are then combined and brought to a final volume of 1 mL with dH₂0.

### 4.3 Cell cultures

The experiments were conducted on both young and senescent BM-MSCs cells, obtained from a 30-year-old Caucasian donor (Promocell). BM-MSCs were cultured in maintenance medium MSC-GM2 (PromoCell) with the addition of Supplement Mix and Penicillin-Streptomycin 1%, in humid atmosphere at 37°C, with 5% CO₂.

### 4.4 Evaluation of the expression levels of osteogenic and pro-inflammatory markers

The pro-osteogenic activity of the individual components (pro-mineralizing and anti-inflammatory) and their combination was firstly determined through the analysis of the modulation of the mRNA levels of the osteogenic marker COL1α1 in young BM-MSCs cells. For the assay setup, young BM-MSCs cells were seeded in 12-wells plates, with a density of 3000 cells/cm² and treated with maintenance medium, with the addition of both individual components and their combination. The young BM-MSCs cells were grown in cell medium added with the pro-mineralizing component (9.15 µg/ml of acid orthosilicic acid and 0.06 µg/ml of vitamin K2), the anti-inflammatory component (0.36 µg/ml of curcumin, 3.9 µg/ml polydatin, 0.15 µg/ml quercetin) or their combination for 7 days. The cells grown in maintenance medium without the substances were used as a negative control. At the end of the treatments, after a short washing in PBS, the cells were lysed directly on plate, with the lysis buffer of the total RNA extraction kit (Norgen Biotek Corporation, Canada). After the extraction process and quantization of the RNA with the Nanodrop instrument, RNA was reverse transcribed using the Takara Bio Inc. kit and the obtained cDNAs were used to perform the RTqPCR assays. Furthermore, the pro-mineralizing and anti-inflammatory activity of the individual components and their combination was determined through the analysis of the modulation of the mRNA levels of the osteogenic marker ALP and of the pro-inflammatory markers IL-1β and MCP-1 in senescent BM-MSCs cells. For the assay setup, the pre-senescent BM-MSCs cells were cultured in maintenance medium supplemented with 0.36 µg/ml of curcumin+3.9 µg/ml of polydatin+0.15 µg/ml of quercetin or with 9.15 µg/ml orthosilicic acid+0.06 µg/ml vitamin K2 or with their combination (using all the five molecules) for 48h. Cells grown in medium maintenance without the substances were used as a negative control. After the treatment, the cells were lysed directly on plate, with the lysis buffer of the total RNA extraction kit (Norgen Biotek Corporation, Canada). After the extraction process and quantization of the RNA with the Nanodrop instrument, RNA was reverse transcribed using the Takara Bio Inc. kit and the obtained cDNAs were used to perform the RTqPCR assays. **Table 10** shows the list of primers used for the RTqPCR experiments, utilizing the SYBR-Green (Takara Bio Inc.).

**Table 10. Sequence of primers used for RTqPCR assays.**

| **Gene** | **Sequence** | **Sequence ID** |
|---|---|---|
| IPO8-F | CGTTCCTCCTGAGACTCTGC | 1 |
| IPO8-R | GAATGCCCACTGCATAGGTT | 2 |
| COL1α1-F | CCAAATCTGTCTCCCCAGAA | 3 |
| COL1α1-R | TCAAAAACGAAGGGGAGATG | 4 |
| ALP-F | GAGAAGCCGGGACACAGTTC | 5 |
| ALP-R | CCTCCTCAACTGGGATGATGC | 6 |
| IL-1β-P | AGATGATAAGCCCACTCTACAG | 11 |
| IL-1β-R | ACATTCAGCACAGGACTCTC | 12 |
| MCP-1-F | GGCTGAGACTAACCCAGAAAAG | 17 |
| MCP-1-R | GTGCCCATGCTACATTTGCC | 18 |

### 4.5 Results

### Evaluation of the pro-mineralizing and anti-inflammatory activity of the combination of the five tested compounds

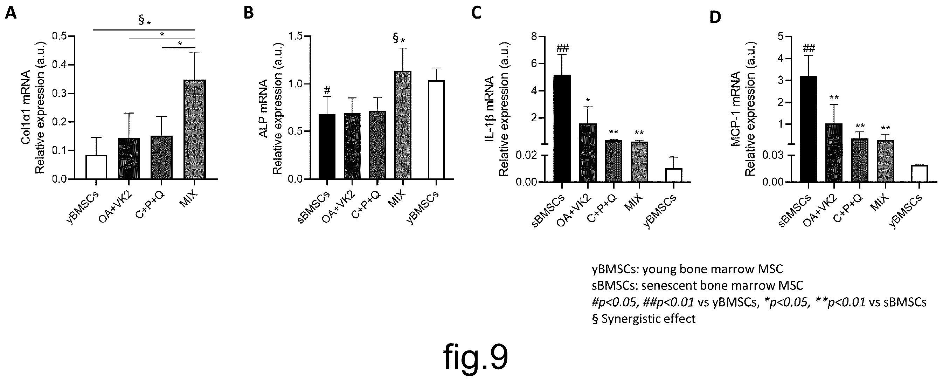

**Figure 9** shows that the pro-mineralizing and the anti-inflammatory components, when used individually, induced only a slight increase of COL1α1 mRNA levels in young BM-MSCs cells, while the treatment with the combination of vitamin K2, orthosilicic acid, curcumin, polydatin and quercetin induced a statistically significant and **synergistic** increase of the COL1α1 expression levels **(****Figure 9A****).** In fact, the treatment with orthosilicic acid and vitamin K2 induced an increase of 68% of COL1α1 mRNA levels, the treatment with curcumin, polydatin and quercetin of 79% and the treatment with the combination of the five molecules of 310%, a value higher than the sum of the effects obtained with the pro-mineralizing and the anti-inflammatory components. **Figure 9B** shows that in senescent BM-MSCs cells there was a significant decrease of ALP expression levels when compared with young BM-MSCs cells and only the MIX treatment (orthosilicic acid+vitamin K2+curcumin+polydatin+quercetin) was able to induce a significant and **synergistic** increase of the mRNA levels of the osteogenic marker ALP. In fact, the treatment with orthosilicic acid and vitamin K2 induced an increase of 11% of ALP mRNA levels, the treatment with curcumin, polydatin and quercetin of 10% and the treatment with the combination of the five molecules of 87%, a value higher than the sum of the effects obtained with the pro-mineralizing and the anti-inflammatory components. Noteworthy, **Figure 9C****,D** show that there was a significant and remarkable increase of both IL-1β and MCP-1 expression levels in senescent BM-MSCs cells when compared with young BM-MSCs, indicating the establishment of a pro-inflammatory state. Our results show that all the treatments were able to significantly decrease the mRNA levels of the pro-inflammatory markers IL-1β **(****Figure 9C****)** and MCP-1 **(****Figure 9D****).**

### 4.6 Conclusions

The combination of orthosilicic acid, vitamin K2, curcumin, polydatin and quercetin exerted **synergistic** effects regarding the increase of the expression levels of the pro-osteogenic marker COL1α1 in young BM-MSCs and the increase of the expression levels of the osteogenic marker ALP in senescent BM-MSCs, demonstrating the **synergistic pro-mineralizing** effects of the combination of these five molecules. In conclusion, the data obtained from the inventor in relation to the pro-mineralizing and anti-inflammatory activities of the combination of orthosilicic acid, vitamin K2, curcumin, polydatin and quercetin indicate that the composition of the present invention represents an effective treatment against the osteopenia and osteoporosis pathological conditions.

### References

1.Bachstetter, A. D., and Van Eldik, L. J. The p38 map kinase family as regulators of proinflammatory cytokine production in degenerative diseases of the CNS; Aging Dis; 2010
2.Christakos S. Recent advances in our understanding of 1,25-dihydroxyvitamin D3 regulation of intestinal calcium absorption; Arch Biochem Biophys; 2012
3.Infante, A., and Rodriguez, C. I. Osteogenesis and aging: lessons from mesenchymal stem cells; Stem Cell Res. Ther; 2018
4.Jin ZH et al., Zoledronic acid accelerates osteogenesis of bone marrow mesenchymal stem cells by attenuating oxidative stress via the SIRT3/SOD2 pathway and thus alleviates osteoporosis; European Review for Medical and Pharmacological Sciences; 2020
5.Jurkic, L.M. et al. Biological and therapeutic effects of ortho-silicic acid and some ortho-silicic acid-releasing compounds: New perspectives for therapy; Nutr. Metab. (Lond.); 2013
6.Lencel, P., and Magne, D. Inflammaging: The driving force in osteoporosis? ; Med. Hypotheses; 2011
7.Lin, Z. et al. Polydatin Ameliorates Osteoporosis via Suppression of the Mitogen-Activated Protein Kinase Signaling Pathway; Front. Cell Dev. Biol.; 2021
8.Madkour, M. M. et al. Current status and future prospects of p38α/MAPK14 kinase and its inhibitors; Eur. J. Med. Chem; 2021
9.Mori, Y. et al., Modeling and simulation of bone mineral density in Japanese osteoporosis patients treated with zoledronic acid using tartrate-resistant acid phosphatase 5b, a bone resorption marker; Osteoporos. Int.; 2018
10. Mulholland, B. S. et al. Monocyte Chemoattractant Protein-1 (MCP-1/CCL2) Drives Activation of Bone Remodelling and Skeletal Metastasis; Curr. Osteoporos; Rep. 2019
11.Oh J.H. et al., Effect of Quercetin-3-O-β-D-galactopyranoside on the Adipogenic and Osteoblastogenic Differentiation of Human Bone Marrow-Derived Mesenchymal Stromal Cells; IJMS; 2020
12.Raisz, L.G. Pathogenesis of osteoporosis. Concepts, conflicts, and prospects; J.Clin. Invest.; 2005
13.Rutkovskiy, A. et al. Osteoblast Differentiation at a Glance; Med. Sci. Monit. Basic Res; 2016
14. Xu, L. et al., Connectivity Map Analysis Identifies Fisetin as a Treatment Compound for Osteoporosis Through Activating the PI3K-AKT Signaling pathway in mouse Pre-osteoblastic MC3T3-E1 cells; Curr. Pharm. Biotechnol.; 2021
15. Zhou, P. et al. Establishing a deeper understanding of the osteogenic differentiation of monolayer cultured human pluripotent stem cells using novel and detailed analyses; Stem Cell Res. Ther.; 2021

## Claims

1. A pharmaceutic or dietary food composition comprising the combination of the active ingredients orthosilicic acid, vitamin K2, polydatin, Curcuma longa extract and quercetin.

2. The composition according to claim 1, which is an oral preparation.

3. The composition according to claim 2, which is an oral, solid or liquid pharmaceutical form, preferably a tablet, a soft or hard capsule, a pill, a powder, a granulate, or a solid controlled release form.

4. The composition according to any of claims 1 to 3, which comprises from 5 to 50 mg of orthosilicic acid per dosage unit.

5. The composition according to anyone of claims 1 to 4, which comprises from 50 to 200 µg of vitamin K2/Menaquinone-7 (MK-7) per dosage unit.

6. The composition according to anyone of claims 1 to 5, which comprises from 20 to 100 mg of polydatin per dosage unit.

7. The composition according to anyone of claims 1 to 6, which comprises from 50 to 500 mg of Curcuma longa extract per dosage unit.

8. The composition according to anyone of claims 1 to 7, which comprises from 20 to 300 mg of quercetin per dosage unit.

9. The composition according to anyone of claims 1 to 8, for the use in the therapeutic or preventive treatment of osteopenia and/or osteoporosis.

## Patentansprüche

1. Pharmazeutische oder diätetische Lebensmittelzusammensetzung, aufweisend die Kombination der wirksamen Bestandteile Orthokieselsäure, Vitamin K2, Polydatin, Curcuma longa-Extrakt und Quercetin.

2. Zusammensetzung nach Anspruch 1, welche eine orale Zubereitung ist.

3. Zusammensetzung nach Anspruch 2, welche eine orale feste oder flüssige pharmazeutische Form, bevorzugt eine Tablette, eine Weich- oder Hartkapsel, eine Pille, ein Pulver, ein Granulat oder eine feste Form mit kontrollierter Freisetzung ist.

4. Zusammensetzung nach einem der Ansprüche 1 bis 3, welche 5 bis 50 mg Orthokieselsäure pro Dosierungseinheit aufweist.

5. Zusammensetzung nach einem der Ansprüche 1 bis 4, welche 5 bis 200 µg Vitamin K2/Menachinon-7 (MK-7) pro Dosierungseinheit aufweist.

6. Zusammensetzung nach einem der Ansprüche 1 bis 5, welche 20 bis 100 mg Polydatin pro Dosierungseinheit aufweist.

7. Zusammensetzung nach einem der Ansprüche 1 bis 6, welche 50 bis 500 mg Curcuma longa-Extrakt pro Dosierungseinheit aufweist.

8. Zusammensetzung nach einem der Ansprüche 1 bis 7, welche 20 bis 300 mg Quercetin pro Dosierungseinheit aufweist.

9. Zusammensetzung nach einem der Ansprüche 1 bis 8, zur Verwendung in der therapeutischen oder präventiven Behandlung von Osteopenie und/oder Osteoporose.

## Revendications

1. Composition pharmaceutique ou alimentaire diététique comprenant la combinaison des principes actifs acide orthosilicique, vitamine K2, polydatine, extrait de Curcuma longa et quercétine.

2. Composition selon la revendication 1, qui est une préparation orale.

3. Composition selon la revendication 2, qui se présente sous une forme pharmaceutique orale, solide ou liquide, de préférence un comprimé, une capsule à enveloppe molle ou dure, une pilule, une poudre, un granulé, ou sous une forme solide à libération contrôlée.

4. Composition selon l'une des revendications 1 à 3, qui comprend 5 à 50 mg d'acide orthosilicique par unité posologique.

5. Composition selon l'une quelconque des revendications 1 à 4, qui comprend 50 à 200 µg de vitamine K2/Ménaquinone-7 (MK-7) par unité posologique.

6. Composition selon l'une quelconque des revendications 1 à 5, qui comprend 20 à 100 mg de polydatine par unité posologique.

7. Composition selon l'une quelconque des revendications 1 à 6, qui comprend 50 à 500 mg d'extrait de Curcuma longa par unité posologique.

8. Composition selon l'une quelconque des revendications 1 à 7, qui comprend 20 à 300 mg de quercétine par unité posologique.

9. Composition selon l'une quelconque des revendications 1 à 8, pour utilisation dans le traitement thérapeutique ou préventif de l'ostéopénie et/ou de l'ostéoporose.
